# EUROPEAN PATENT SPECIFICATION

(11) **EP 1 827 522 B1**
(45) Date of publication and mention of the grant of the patent: **17.03.2010**
(21) Application number: 05854658.1
(22) Date of filing: 15.12.2005
(51) Int. Cl.: A61L 27/18, A61F 2/16, C08L 83/04

(54) **COMPOSITIONS FOR INJECTABLE OPHTHALMIC LENSES**
ZUSAMMENSETZUNGEN FÜR INJIZIERBARE OPHTHALMISCHE LINSEN
COMPOSITIONS POUR LENTILLES OPHTALMIQUES INJECTABLES

(30) Priority: 20.12.2004 US 638052 P; 20.12.2004 SE 0403091
(43) Date of publication of application: 05.09.2007
(73) Proprietor: AMO Groningen B.V., 9728 NX Groningen (NL)
(72) Inventor: HAITJEMA, Hendrick, Jan, NL-9321 At Peize (NL); DEURING, Hendrik, NL-9403 XZ Assen (NL)
(74) Representative: Holmberg, Martin Tor
(86) International application number: PCT/US2005/045985
(87) International publication number: WO 2006/069012

(56) References cited:
- WO-A-01/76651
- WO-A-93/21245
- WO-A-95/17460
- US-A- 5 444 106
- US-A1- 2003 088 044
- US-B1- 6 432 137

## Description

### RELATED APPLICATION

The present application claims priority under 35 U.S.C. §119 of U.S. Application Serial No. 60/638,052 filed December 20, 2004, and of Swedish Patent Application No. 0403091-2, filed on December 20, 2004.

### FIELD OF THE INVENTION

The present invention relates to the field of ophthalmology, more specifically to surgical treatment of eye disorders, such as cataract and/or presbyopia, by replacing the natural lens with an injectable accommodative intraocular lens. The invention provides an injectable composition comprising a mixture of non-functional and functional polysiloxanes, which composition is cured *in situ* in the capsular bag of the eye, thereby providing a new intraocular lens.

### BACKGROUND OF THE INVENTION

The human eye is a highly evolved and complex sensory organ. It is composed of a cornea, or clear outer tissue, which refracts light rays en route to the iris, the iris, which controls the size of the pupil and thus regulates the amount of light entering the eye, and a lens, which focuses the incoming light through the vitreous fluid onto the retina. The retina converts the incoming light into electrical energy, which is transmitted through the brain stem to the occipital cortex resulting in a visual image. In a perfect eye, the light path from the cornea through the lens and vitreous fluid to the retina is unobstructed. Any obstruction or loss in clarity within these structures causes scattering or absorption of light rays, which results in a diminished visual acuity. For example, the cornea can become damaged, resulting in edema, scarring or abrasions; the lens is susceptible to oxidative damage, trauma and infection; and the vitreous fluid can become cloudy due to hemorrhage or inflammation.

As an individual ages, the effects of oxidative damage caused by environmental exposure and endogenous free radical production accumulate, resulting in a loss of lens flexibility and denatured proteins that slowly coagulate, thereby reducing lens transparency. The natural flexibility of the lens is essential for focusing light onto the retina by a process referred to as accommodation. Accommodation allows the eye to automatically adjust its refractive power for viewing objects at different distances. When the cumulative effects of oxidative damage diminish this flexibility, thus reducing near vision ability, it is known as presbyopia. Presbyopia usually begins to occur in adults during their mid-forties. An individual with presbyopia needs spectacles of different powers for different object distances, or alternatively spectacles or contact lenses that have multifocal or progressive optics. These alternatives have limitations in many practical situations.

Lenticular cataract is a lens disorder resulting from further development of coagulated protein. There are four common types of cataracts: senile cataracts associated with aging and oxidative stress; traumatic cataracts that develop after penetrating or non-penetrating impacts of objects on the eye or following exposure to intense radiation; complicated cataracts that are secondary to diseases such as diabetes mellitus or eye disorders such as detached retinas, glaucoma and retinitis pigmentosa; and toxic cataracts resulting from medicinal or chemical toxicity. Regardless of the cause, the disease results in impaired vision and may lead to blindness.

Treatment of cataract and/or presbyopia requires surgical removal of the lens, involving phacoemulsification followed by irrigation and aspiration. Without a lens, the eye is unable to focus the incoming light onto the retina. Consequently, an artificial lens is used to restore vision. Three types of prosthetic lenses are available: cataract spectacle lenses, external contact lenses and intraocular lenses (IOLs). Intraocular lenses, which can be either monofocal or multifocal, are currently used in the majority of cases to overcome the difficulties associated with cataract spectacle lenses and contact lenses. Multifocal intraocular lenses provide pseudo-accommodation, i.e. both distant and near objects can be seen sharply, however, there is always a reduction of contrast sensitivity in comparison with monofocal lenses. Multifocal lenses are sometimes used in cases of presbyopia without cataract, so-called clear lens exchange, despite the reduction in contrast sensitivity.

IOLs mentioned in the prior art literature usually belong to one of the following categories: rigid, foldable, expansive hydrogels and injectable. The earliest IOLs coming into surgical practice were rigid implants, composed of poly(methylmethacrylate). These types of lenses require a large corneal incision (>5.0mm), which resulted in protracted recovery times and the likelihood of introducing astigmatism. In an effort to reduce recovery time and patient discomfort, several small incision techniques in conjunction with intraocular lenses implantable through these small incisions have been developed.

Present IOLs, which are designed for small incision implantation (3.5-2.5mm), have elastomeric characteristics and are made of soft silicone or acrylic rubbers, or soft hydrogel materials. These types of lenses can be rolled or folded, inserted into the capsular bag, and then unfolded once inside but typically provide no accommodative ability. However, there exist so-called accommodative lenses, which are claimed to provide accommodative ability by moving anteriorly in response to ciliary muscle contraction, but the optical effect is minimal, about 1 diopter change (corresponding to an object distance of 1 meter).

To further develop IOLs and reduce the size of the surgical incisions (1.5-1.0mm), techniques using injectable IOLs have been suggested. In these techniques, a low viscosity lens material is directly injected into the emptied capsular bag and cured *in situ* as a part of the surgical procedure. In such a process, the capsular bag is used as a mold to form the shape of the lens and thereby contribute to the control of its refraction. There have been several attempts to develop materials suitable for use as an injectable lens to replace the natural crystalline lens, and having a viscosity suitable for injection through a standard cannula. The resulting lenses had a Young's modulus below about 10 kPa.

The technique of cataract extraction and replacement of the natural IOLs is disclosed in US patents Nos. 5,278,258, 5,391,590, 5,411,553 and 5,476,515.
Cataract extraction and replacement of the natural lens for an injectable accommodating IOL, i.e. an artificial crystalline lens (ACL) as described for example in WO 01/76651, which is hereby incorporated by reference in its entirety, involves injection of a liquid having sufficiently low viscosity through a small incision into the capsular bag, followed by crosslinking of the liquid to create a lens of the required shape, using the form of the capsular bag as the mold. To reproduce the optical performance of the natural lens, the replacement lens will require a refractive index of about 1.42, and, to respond to the accommodating forces, the compression modulus (Young's modulus) of the lens should be in the range of 1-5 kPa or less. Researchers, e.g. Haefliger et al (1994), J. Refractive and Corneal Surgery 10, 550-555, in the field of ACLs have used silicone-derived systems for filling the capsular bag, either in the form of silicone oils or LTV (low temperature vulcanizing) silicone elastomers. Such systems suffer from certain disadvantages in the context of lens refilling in that lenses resulting from dimethyl silicones exhibit a restricted refractive index (1.40).
Moreover, the LTV silicone elastomers cure slowly; up to 12 hours may be needed to complete their setting. This slow setting results in material loss from the capsular bag through the corneal incision. Alternatively, where a precured material is used to minimize leakage, the quality of the lens' surface is compromised.

In certain circumstances there is a concern that the low molecular weight compounds and/or non-crosslinked fractions, which may migrate (bleed or oil) from a crosslinked polysiloxane network, may affect the surrounding tissues or may change its properties, e.g. if non-crosslinked fractions should migrate from a lens this could cause a decrease in optical quality and/or stiffening of the lens. Further examples come from a different technical field, namely that of breast prostheses, in which the migration is minimized by prepolymerization of the silicone polymers used and by avoiding non-crosslinkable polymer fractions, see US patents Nos. 5,741,877 and 4,558,112.

To form a lens of the correct power from a liquid composition, by molding in the capsular bag, the interior and exterior pressures of bag must be balanced during cure to ensure complete interfacial contact between the polymerizing composition and the internal surface of the bag. Additionally, any detachment of the surface of the lens from the inside of the bag will scatter light. Consistent interfacial contact is best obtained from compositions that cure rapidly following injection into the capsular bag. A further important consideration in this respect, is that the ophthalmologist is able to monitor, at the point of formation, the power of the lens molded during the surgery with confidence.

### SUMMARY OF THE INVENTION

We have found that a very careful design of the starting polymers allows formulating a composition with an improved control of the injection behavior and the properties of the resulting elastomeric intraocular lens, and thus the present invention provides an ophthalmic injectable composition composed of components such that the final crosslinked product obtained has a minimum of migratable species commensurate with the attainment of the required elasticity.

In one embodiment, the present invention provides an injectable ophthalmic composition, suitable for forming an intraocular lens in the capsular bag of the eye from which an impaired natural lens has been surgically extracted. The composition comprises linear non-functional polysiloxane, linear terminally functional polysiloxane, and at least one crosslinker, wherein the linear terminally functional polysiloxane comprises a mixture of linear terminally monofunctional polysiloxane and di-functional polysiloxane. In a specific, non-limiting embodiment, the functionality of the polysiloxanes is provided by unsaturated groups and the at least one crosslinker is a multifunctional crosslinker.

In another embodiment, the present invention provides an intraocular lens comprising an inventive composition. In a specific embodiment, the lens is able to accommodate, as does a natural lens, to provide the individual with near and far vision. In a further embodiment, the intraocular lens comprises a minimum of migratables/extractables without having a reduced elasticity.

Other embodiments and advantages of the present invention will be readily appreciated, as the same becomes better understood, after reading the subsequent detailed description and the claims.

### DETAILED DESCRIPTION OF THE INVENTION

Within the present disclosure, the following definitions apply:
By the term "injectable composition", as used herein, is meant a composition having a suitable viscosity to be readily injected through a conventional cannula, which has an 18 Gauge needle dimension or finer dimensions. In a more specific embodiment, a composition according to the invention is able to pass through a 21 Gauge needle. To comply with these criteria of injectability, the composition according to the present invention should have a viscosity less than about 60,000 cSt. In a specific embodiment, the viscosity is less than about 8000 cSt.
By the term "ophthalmic composition", as used herein, is meant a composition suitable for ophthalmic use.
By the term "intraocular lens", or "IOL", as used herein, is meant an artificial lens, which is arranged, or suitable to be arranged, in the capsular bag of an eye, from which the natural lens has been extracted.

The technique of cataract explantation and lens replacement for an artificial crystalline lens, ACL, involves the metered injection of a low viscosity liquid as described, through a small incision (≈1 mm diameter), into the capsular bag, followed by its polymerization to create a lens of the required shape, using the form of the capsular bag as the mold. To reproduce the optical performance of the natural lens, the replacement lens will require a refractive index close to that of the natural lens (about 1.41-1.42) and to respond to the accommodating forces the Young's modulus will be in the range 1-5 kPa. An accommodative capsular lens is thus an IOL formed by filling the capsular bag with the precursors of an elastomer, and causing, or allowing, the elastomer to set in the form of the natural lens. Said lens is able, due to its pliability, to provide accommodative ability under the influence of the ciliary muscle.

In the present invention the term "polysiloxane" is intended to mean polymer having at least one siloxane unit, and includes one or more of such polymers.

In the present invention the terms "extractables" or "migratables" are intended to mean low molecular weight compounds that can either migrate, i.e. bleed, from the polymer network or be extracted from the polymer network, and which may thus diffuse through or otherwise pass from the capsular bag into the surrounding vitreous fluid.

The term "polymer network" as used herein is intended to mean cured compositions of one or more polymers, for example when a composition according to the present invention is cured, it will form a polymer network.

By the term "linear polysiloxane", as used herein is meant a non-branched polysiloxane. It is understood that the term also encompasses essentially linear polysiloxane, wherein only a small fraction of the polysiloxane is branched.

The term "functional", as used herein, refers to the property of being reactive under suitable conditions. Further, in this field of polymers, a functional group is a group selected such that it is able to combine with a like or a specific dissimilar group to bring about or to cause chain extension or crosslinking.

The term "terminally functional", as used herein, identifies that the functionality, i.e. the functional group, is present at the end of a polymer's chain. Thus, a linear polymer has two ends, and so a terminally functional linear polymer may be either "monofunctional" or "difunctional". Reaction of the difunctional polymers with crosslinkers bearing reactive groups (curing) provides a crosslinked network.

Polymers lacking any functional groups are herein referred to as "non-functional polymers", and are incapable of participating in the chemical formation of the network. Despite this terminology, these polymers have a physical function of great importance in the molded intraocular lens, since their presence reduces the modulus of the lens.

By the term "dangling chain" as used herein is intended to mean an end of a polymer chain that is free to participate in free rotational movement and conformation change and is restricted only in translational displacement from the polymer network. A dangling chain is formed where a terminally monofunctional linear polymer molecule participates in the curing reaction so that the functional end-group is bound to the polymer network, allowing the other end of the captured polymer's chain to move in the polymer network as described above. The polymer free end constitutes a dangling chain and it functions as a plasticizer within the cured network.

In the present disclosure, the term "mixed-end blocker polymers" (MEBP) is intended to mean a mixture comprising linear polymers having two reactive ends, polymers having one reactive end and one non-reactive end and polymers having two non-reactive ends.

By the term "multifunctional crosslinker" as used herein is meant a crosslinker having more than three reactive sites. A crosslinker comprises points where the polymer chains can be linked.

By the term "siloxane crosslinker" as used herein is meant that the crosslinker comprises at least one Si-atom.

By the term "crosslinking agent" as used herein is meant a compound that will start the crosslinking process, e.g. a catalyst.

In one embodiment, the present invention provides an ophthalmic composition comprising a mixture of linear terminally di-functional and monofunctional polysiloxanes and linear non-functional polysiloxanes and at least one crosslinker, which composition when crosslinked comprises a minimum of extractables, suitable for forming a lens. In a specific embodiment, the ophthalmic compositions according to the invention contain less than about 50% by weight of extractables, more specifically less than about 40% by weight of extractables, and even more specifically less than about 30% by weight of extractables. In a further embodiment, the ophthalmic compositions contain less than about 20% by weight of extractable components. The advantage of having a composition comprising linear terminally monofunctional polysiloxanes is that the polysiloxanes will have three roles, they will extend the polymer network, by forming the dangling chains, which act as plasticizers for the polymer network and provide an enhanced solubilizing capacity for the non-functional polysiloxanes within the polymer network. Due to these characteristics, the amount of extractables is reduced without increasing the modulus of elasticity of the network above that required of an accommodating lens. The present invention produces a cured composition with a modulus matching that of the natural lens material which has a Young's modulus of 1-5 kPa. This control is effected as a consequence of the substitution of free polysiloxane oil by dangling siloxane chains attached to the polymer network. Thus, the obtained polymer network according to the present invention will have less extractables due to the reduced amounts of linear non-functional polymers and due to the better dissolving capacity of the polymer network lowered migratables, but the network will have very low modulus.

A further reduction of extractables is also possible if the non-functionalized polysiloxanes used are fractionated in order to remove low molecular weight polymers. A method for fractionating polymers is disclosed in International Patent Application WO 03/062282, which is hereby incorporated by reference.

Those skilled in the art will appreciate that said fractionation, when applied to functionalized polysiloxanes, will give new possibilities for the modification of the mechanical properties of the polymer networks since the molecular weight between the crosslinks will be increased and/or the effectiveness of the dangling chains optimized. Additionally, by removing low molecular weight polymers, the ability of such materials to escape the capsular bag is reduced.

According to one specific embodiment of the present invention, the crosslinker is a multifunctional crosslinker. In one embodiment, the multifunctional crosslinker is a polymeric crosslinker having an average of 5-9 reactive sites. In a more specific embodiment, the multifunctional crosslinker is a siloxane crosslinker which comprises organohydrogen siloxane and dialkyl siloxane, for example, a copolymer of methylhydrosiloxane and dimethylsiloxane. The advantage of using a multifunctional crosslinker, i.e. of using a crosslinker having a higher degree of functionality than usually used, is that more polymers can be bonded to the obtained polymer network according to the present invention while this network still will retain the desired softness due to the dangling ends of the above-mentioned monofunctional polysiloxanes. The network obtained according to the present invention gives the artificial lens material mechanical dynamics comparable to those of the human lens, i.e., it has a G'/G" ratio similar to that of the natural lens material (where G' is the elastic shear modulus and G" is the viscous shear modulus at 1Hz), due to the combination of the multifunctional crosslinker and polysiloxanes of different functionality. Hence, the present invention provides a plasticized polysiloxane network that matches most closely the mechanical behavior of the natural lens and so responds correctly in the eye to the accommodating forces.

According to another embodiment according to present invention, the functionality of the linear terminally functional groups is provided by unsaturated groups. The preferred unsaturated functional groups are vinyl (thermal curing) or acrylic groups (photo curing). However, one of ordinary skill in the art will be aware of other suitable groups falling within the scope of the present invention. Thus, when a composition according to the present invention is thermocurable, it also comprises a catalyst, which is for example a platinum complex, more specifically a complex between platinum and a siloxane-containing compound (hydrosilylation catalyst), and preferred is a complex between platinum and divinylmethylsiloxane. One catalyst for use in the present invention may be prepared according to the method disclosed in the examples. A solvent which may be used for the catalyst solution is a low molecular weight solvent. However, this solution is added to the thermocurable compositions according to the present invention in such low amounts that it will not affect the over all properties of the compositions. In one embodiment, the solvent used is preferably a di-functional polymer having the same composition as the polymers contained within the polymer network since this will maximize the chance of a reaction while minimizing phase separation. The functional groups, for example the vinylic groups, of the functional polysiloxanes will react with the silicone bonded hydride (Si-H) groups of the crosslinker (multifunctional) in the presence of the catalyst (as is e.g. disclosed in WO 00/22454, which is hereby incorporated by reference). General references for the crosslinking process are US patents No. 5,278,258 and 5,444,106, which are hereby incorporated by reference. One of ordinary skill in the art can also identify a large number of different alkenyl moieties and different routes of how to synthesize functional polysiloxanes, e.g. vinyl functional polysiloxanes, thus this will not be discussed in detail.

However, when a composition according to the present invention is photocurable, it comprises also a photoinitiator, which can either be incorporated in or bonded to the crosslinker (as disclosed in e.g. WO 00/22460, which hereby is incorporated by reference). Examples of suitable functional acryl groups include acrylamidopropyl, methacrylamidopropyl, acryloxyhexyl and methacryloxyhexyl. Preferably, the functional acryl groups are attached to the terminal ends of polysiloxane molecules. Those skilled in the art can consider numerous such alternatives, which maintain the basic function of having an acryl group for subsequent crosslinking/curing of the polysiloxane molecules into a larger network together with a photoinitiator. In the same manner, it is also understood that the meaning of acryl group should include acryl or substituted acryl, such as methacryl, moieties attached through a variety of linkages including ester, amide and urethane linkages, or functional analogues of acryl capable of undergoing crosslinking reactions with a photoinitiator. The photoinitiators employed according to the present invention are medically acceptable, photobleaching and are preferably activated in the visible range, including blue light activated photoinitiator types, e.g. derived from acyl phosphine oxides and bisacylphosphine oxides and titanocene photoinitiators. Suitable photoinitiators for injectable lens forming compositions are also discussed in WO 99/47185 and in WO 00/55212, which are both incorporated herein by reference. The photoinitiator may be a conjugate of a photoactive group and a macromolecule capable of participating in a crosslinking reaction with acryl-terminated polysiloxanes.

The compositions according to the present invention can also comprise compounds and/or additives usually used in injectable ophthalmic compositions, for example UV absorbers, etc. and the non-functional polysiloxanes in the inventive compositions (both thermocurable and photocurable) can, for example, have trimethylsiloxane end groups.

According to another specific embodiment of the present invention, the molar ratio between the linear terminally di-functional polysiloxane, the linear terminally monofunctional polysiloxane and the linear non-functional polysiloxane is about 0.5-1:1-2:0.5-1. In a more specific embodiment, the molar ratio is about 1:2:1.

According to yet a further embodiment of the present invention, the linear terminally functionalized polysiloxanes and the non-functional polysiloxanes are essentially composed of the same monomer (as defined below), i.e. they are compatible with each other. Thus, they are completely miscible and form stable solutions under the conditions present in the eye in the crosslinked network composition used to form the ACL. This feature is important for ophthalmic applications, such as IOLs, since structural differences in the siloxane polymers, leading to phase separation of the constituent parts, may cause scattering of light within the lens. This will be observed as haziness, mistiness or opacification within the lens, undesirably diminishing the vision of the patient.

Most preferably, the linear functional and non-functional polysiloxanes according to the present invention comprise the same random tercopolymer. Preferably, both types of polysiloxane are synthesized in the same reaction (a so-called "one-pot synthesis") in which monomers and end-capping reagents are mixed and reacted, thus a mixture of end-capped polysiloxanes having essentially the same composition as the polysiloxane network's precursory formulation is obtained. The possibility of making both the component polysiloxanes, simultaneously and together, according to the present invention is very advantageous since their compatibility is ensured and their preparation simplified.

In another embodiment of the present invention, the linear terminally functional polysiloxane and non-functional polysiloxanes of the present invention comprise essentially the same monomer units, which have the general formula of -RₐR_{b}SiO-. Prefereably, Rₐ and R_{b} are the same or different, substituted or unsubstituted alkyl or aryl groups which are bonded directly to the silicon atom. One of the alkyl or aryl groups may be substituted with at least one fluorine atom. More preferably, Rₐ, in at least one of the monomer units, is fluoroalkyl and R_{b} in that monomer unit is alkyl. In a specific embodiment, Rₐ is 3,3,3-trifluoroalkyl and the obtained polysiloxanes have a specific gravity greater than about 1. In order to provide the polysiloxanes with the typically high specific gravity, it is preferred that the fluoroalkyl containing monomers exceed about 4 mol% of the polymer. An additional specific form of siloxane monomer units is an arylsiloxane and, more specifically, arylsiloxane monomer units comprising diphenylsiloxane and phenylalkylsiloxane.

According to a preferred embodiment of the present invention, the linear terminally functional and non-functional polysiloxanes are derived from three different siloxane monomers having the general formula (R₁R₂SiO)₁, (R₃R₄SiO)ₘ and (R_{S}R₆SiO)ₙ. One of the three monomers has a specific gravity greater than about 1.0 and R₁ and R₂ are the same or different substituted or unsubstituted C₁₋₆ alkyl groups, R₃ and R₄ are the same or different substituted aryl or C₁₋₆ alkyl groups, and R₅ and R₆ are the same or different fluoroalkyl or alkyl groups, and 1 is in the molar fraction range of 0 to 0.95, m is in the molar fraction range of 0 to 0.7 and n is in the molar fraction range of 0 to 0.65. In specific embodiments, R₁ and R₂ are methyl, R₃ is phenyl and R₄ is phenyl or methyl, R₅ is trifluoropropyl and R₆ is methyl and it is also preferred that the polysiloxanes obtained are, as mentioned above, random terpolymers. Alternatively, the polysiloxanes can be higher polymers than terpolymers, including but not limited to tetrapolymers with the same monomer types as mentioned above.

In one embodiment, the polysiloxanes comprise at least about 4 mol% of trifluoropropylmethyl siloxane and 1 to 50 mol% of diphenylsiloxane and/or methylphenylsiloxane. More preferably, the polysiloxanes comprise about 4 to 65 mol% 3,3,3 trifluoropropylmethyl siloxane, 1 to 50 mol% of diphenylsiloxane and dimethylsiloxane monomer units. One suitable specific polysiloxane composition according to the present invention for injection into the capsular bag of the human eye for the formation of ACL comprises about 10.0 mol% trifluoropropylmethyl siloxane, about 6.9 mol% diphenylsiloxane and about 82.6 dimethyl siloxane monomer units. Small amounts of endblockers are also used for synthesizing the composition (as mentioned above, e.g. is 0.5 mol % of divinyltetramethyldisiloxane + hexamethyldisiloxane). Other suitable polysiloxanes, which can be used according to the present invention, are well known in the field; see for example WO 00/22459, WO 00/22460 and WO 01/76651, incorporated herein by reference.

The compositions defined in the preceding section are designed to meet the often conflicting requirements of an injectable ACL. The refractive index of the final implanted lens is regulated by the selection of the siloxane monomer composition of the polysiloxane from which it derives. It is to be understood that the refractive index, if this is required for a specific optical application (see below), can be up to about 1.45 within the context of the present application. The polysiloxanes according to the present invention are, also, suitable for the preparation of an intraocular lens by a crosslinking reaction. To facilitate injection and minimize leakage of polymer from the eye during injection, these suitable polysiloxanes have, as mentioned before, a specific gravity of greater than about 1 which prevents floatation of the injected composition, and a viscosity suitable for injection through a standard cannula. Most preferably, the polysiloxanes have a specific gravity of from about 1.03 to about 1.20.

According to another embodiment of the present invention, the viscosity of the non-functional polysiloxanes does not exceed the viscosity of the terminally functional polysiloxanes, thus one fraction of the polysiloxanes prepared to be included in the composition can be provided with functional groups (e.g. vinyl end-capped), while the other fraction is included in its non-functional form. In one embodiment, the polysiloxanes have a molecular weight which is sufficiently high to avoid loss from the capsular bag, for example by diffusion. By conducting tests on human capsular bag tissue, it has been found that the non-functional polysiloxanes preferably have a number average molecular weight (Mₙ) exceeding a value of about 5000 Dalton (D) to substantially reduce the risk of diffusion of such polysiloxanes through the capsular bag. In a more specific embodiment, the molecular weight exceeds 7000 D, and in a more specific embodiment, the molecular weight exceeds about 10,000 D. The non-functional polysiloxanes preferably have a sufficiently high molecular weight so as to substantially prevent diffusion from the polymer network. In accordance with the present invention, it has also been found that a suitable Young's modulus is obtainable with the inventive polysiloxane compositions after a crosslinking process with monofunctional and di-functional polysiloxanes and at least one multifunctional crosslinker, in the presence of non-functional polysiloxanes.

Thus, according to a specific embodiment of the present invention, the polysiloxanes are poly(dimethyl-co-diphenyl-co-trifluoropropylmethyl)siloxanes having a molecular weight, Mₙ, in the range of 10,000 to 25,000 D, a refractive index of 1.40 to 1.45, and a density greater than about 1.

Furthermore, according to another embodiment of the present invention, the compositions can also comprise another crosslinker, which is also a multifunctional crosslinker. This crosslinker is preferably an organosiloxysilane (organohydrogen siloxane), most preferably the said crosslinker is tetrakis(dimethylsiloxy)silane (see US Patents No. 5,278,258 and 5,444,106 which are hereby incorporated by reference). In a specific embodiment, the multifunctional crosslinker may comprise a mixture of a tetrakis(dimethylsiloxy)silane and a trikis(dimethylsiloxy)silane partly modified with a UV-absorbing group. By this device a UV-absorbing moiety is introduced in a controlled and permanent way into the ultimate siloxane network.

The amounts of the components of the injectable material can be varied in accordance with specific conditions which are desired or encountered. For example, it is desirable to have a reasonably fast (60-90 min) thermal curing process at ambient body temperature, and for an injected composition to be gelled into a stable polymeric network within a suitable working time for a surgeon. One of ordinary skill in the art will be able to find suitable variations of the amount of the components and selecting suitable crosslinkers to obtain a suitable crosslinking density. In a preferred composition according to the invention, the molar ratio of Vinyl/SiH groups= 1.00/(0.50-1.00).

Another aspect of the present invention provides a composition suitable for intraocular lenses having good injectability, sufficiently low modulus to be able to accommodate, appropriate balance between elasticity (G') and viscous behavior (G") for the right mechanical response to eye accommodative muscle action, and a low amount of extractables.

According to another specific aspect of the invention an intraocular lens comprises any one of the inventive compositions described herein. In a specific embodiment, the lens is an accommodative lens, most preferably an artificial crystalline lens. Thus, in order to restore the refractive index of the natural lens (about 1.42), the polysiloxane compositions according to the present invention are capable of providing lenses having refractive indices in the range of about 1.40-1.45. Preexisting ametropia, i.e. refractive error, can be corrected by choosing another refractive index in the range available, about 1.40-1.45, obviating the need to wear any refractive correction following implantation of an ACL.

According to another aspect of the invention, a method, in which the capsular bag is used as a mold, of producing an intraocular lens in the capsular bag of an eye in a patient in need thereof, comprises the step of:
(i) extracting the current lens from the capsular bag;
(ii) injecting a composition according to the present invention into the capsular bag; and
(iii) causing the composition to cure into an intraocular lens.

The method is also used for producing an accommodative intraocular lens and an artificial crystalline lens.

### EXAMPLES

The following examples are included in order to illustrate the principles of the present invention and should not in any way be interpreted as limiting the scope of the invention. Further, it is to be understood by the skilled person that the claimed compositions are prepared by mixing a formulation of polysiloxane and catalyst with a formulation of the crosslinker(s) just prior to its use. It is also to be understood that the compositions according to the present invention can comprise further conventional constituents, such as crosslinkers for effecting curing and that as a secondary role these may be used, as is customary in silicone IOLs, for the introduction of UV-Vis (blue light) light absorbers.

It is clear to a person of ordinary skill in the art that branched polymers may also be used. However, in the technology of preparing injectable and accommodative silicone intraocular lenses, linear polysiloxanes of controlled functionality, structure, degree of branching, and molecular weight are required, to produce formulations with the necessary rheological characteristics, pre- and post-gelation, and the gelation profile, together with the optimal mechanical and low-migratory properties, and the requisite refractive index in the gelled state, which can only be obtained from networks with a well-defined structure.

### Brief description of the Examples

Examples 1-3 disclose the preparation of examples of preferred polymers comprised in the compositions according to the present invention in a specific ratio of functionalities of 25 % divinyl: 50% monovinyl :25% non-vinyl. The difference between the examples is that the polymers obtained have different refractive indices.

Example 4 discloses the preparation of a polymer that will be used for the preparation of the platinum complex catalyst solution.

Example 5 discloses the preparation of one of the crosslinkers used in the compositions according to the present invention comprising two multifunctional crosslinkers.

Example 6 discloses the preparation of cured compositions according to the invention.
The components added to the compositions according to the present invention have different functions. The platinum catalyst in the form of a synthesized 1,3-divinyltetramethyldisiloxane complex will initiate and propagate the thermal curing. The multifunctional copolymeric crosslinker, methylhydrosiloxane dimethylsiloxane, will crosslink the prepolymers of siloxane. The retarder 1,3-divinyl tetramethyldisiloxane will decrease the curing to a preferred gel time/curing time. The UV-absorber, coded UV-QXL2, protects the retina from UV-radiation and is introduced and is connected to the polymeric network, to ensure its permanence, by using the partly modified tetrakis(dimethylsiloxy)silane crosslinker, which combines the crosslinking functions and a LTV absorbing group.

The shear complex moduli (G*) and ratios G'/G" of shear storage moduli (G') and shear loss moduli (G"), which are a measure of the softness and the mechanical dynamics of the cured formulations, are measured, to ensure a behavior similar to that of the natural human lens material.

Since they are made in a single reaction step, it is impossible to directly confirm the precise composition, i.e., the molar ratios of di-, mono-vinyl, and non-functional polymers, of the MEBP polysiloxanes, instrumentally. Their compositions are estimated, instead by making comparisons of their rheology with the rheology of mixtures of known vinyl-terminated silicone polymer compositions.

### Example 1: Preparation of a mixed-end blocker siloxane polymer (MEBP) by using 50 wt% divinyl-terminated end blockers (DVTEB) and 50 wt% non-vinyl-terminated end blockers (NVTEB) and having a refraction index (RI) of 1.43

To a dry 2000 ml flask are weighed in order: octaphenylcyclotetrasiloxane (182.9 g, 0.231 moles), 3,3,3-trifluoropropylmethylcylclotrisiloxane, (205.9 g, 0.439 moles), dimethylcyclotetrasiloxane, (725.4 g, 2.45 moles), α,ω-divinyldimethylsiloxane oligomer end-blocker (42.97 g), and trimethyldimethylsiloxane oligomer end-blocker (43.0g). The reagents are dried under vacuum, their mixture is heated under reflux at 80°C for 30 minutes, and purged with nitrogen, and potassium silanolate (0.54g, 1.37 mmoles) is added. The temperature was increased to 135°C and the mixture is heated and stirred for 2 hours followed by 18 hours at 155°C. After cooling, the product is diluted with dichloromethane (840 ml) and washed with HCl (0.0007M, 840 ml), with HCl (0.0014M, 840 ml), twice with water (2X840ml), twice with a methanol/tetrahydrofuran mixture (7/3, 2X840ml), and finally twice with methanol (2X840ml). The solvent is then removed by heating at 100°C under a vacuum < 1 mbar for several hours. The polysiloxane product is colorless, with refractive index 1.4280 (at 589.6nm at 25°C). Viscosity at 25°C is 1588cP. ¹H-NMR, 300MHz, gives unit mole ratios: dimethyl / diphenyl / trifluoropropyl / divinyltetramethyl + hexamethyl of 0.826 / 0.069 / 0.100 / 0.00542. GPC gives M_{w} 20580 D.

### Example 2: Preparation of a mixed-end blocker siloxane polymer by using 50 wt % DVTEB and 50 wt% NVTEB, having a RI of 1.40

The polymerization method of Example 1 is repeated using a different ratio of monomers. Octaphenylcyclotetrasiloxane,(30.72 g, 0.04 mol) 3,3,3-trifluoropropylmethylcylclotrisiloxane (349.18 g, 0.75 mol), dimethylcyclotetrasiloxane (733.73 g, 2.47 mol), α,ω-divinyldimethylsiloxane oligomer end-blocker (42.88 g), trimethyldimethylsiloxane oligomer end-blocker (42.99 g ), and potassium silanolate (0.55 g). The purification and vacuum stripping processes of Example 1 are repeated to isolate the colorless product which has a refractive index of 1.4000 (589.6nm at 25°C), and a viscosity at 25°C of 1015cP. ¹H-NMR, 300MHz, gives unit mole ratios: dimethyl / diphenyl / trifluoropropyl / divinyltetramethyl + hexamethyl of 0.806 / 0.012 / 0.177 / 0.00503. M_{w} is determined by ¹H-NMR as 19857 D.

### Example 3: Preparation of mixed end-blocker siloxane polymer using 50 wt% DVTEB + 50 wt% NVTEB of RI 1.45

The polymerization method of Example 1 is repeated with a different ratio of monomers, employing octaphenylcyclotetrasiloxane (305.37 g, 0.385 mol), 3,3,3-trifluoropropylmethylcylclotrisiloxane (97.73 g, 0.209 mol), dimethylcyclotetrasiloxane (712.95 g, 2.404 mol), α,ω-divinyldimethylsiloxane oligomer end-blocker (43.00 g), trimethyldimethylsiloxane oligomer end-blocker (42.99 g), and potassium silanolate (0.55 g). The purification and vacuum stripping processes of Example 1 are repeated to isolate the colorless product which has a refractive index of 1. 4504 (589.6nm at 25°C), and a viscosity at 25°C of 2568cP. ¹H-NMR, 300MHz, gives unit mole ratios: dimethyl / diphenyl / trifluoropropyl / divinyltetramethyl + hexamethyl of 0.835 / 0.115 / 0.045 / 0.00477. GPC gives M_{w} 17737 D.

### Example 4: Preparation of DVT siloxane polymer solvent for the platinum catalyst (lower MW) of RI 1.43.

The polymerization method of Example 1 is repeated on a 600 g reagent scale with the use of DVTEB, employing octaphenylcyclotetrasiloxane (91.4g, 0.115 moles), 3,3,3-trifluoropropylmethylcylclotrisiloxane (103.0g, 0.22 moles), octamethylcyclotetrasiloxane (251.2g, 0.849 moles), α,ω-divinyldimethylsiloxane oligomer end-blocker (154.5g, 92.0 mmoles), and potassium silanolate (0.27g, 0.683 mmoles). The purification and vacuum stripping processes of Example 1 are repeated to isolate the colorless product which has a refractive index of 1.428 (589.6nm at 25°C), and its viscosity at 25°C is 364cP. ¹H-NMR, 300MHz, gives unit mole ratios: dimethyl / diphenyl / trifluoropropyl / divinyltetramethyl + hexamethyl of 0.804 / 0.068 / 0.099 / 0.02847. GPC gives Mₙ 6,644 D and M_{w} 10,224 D.

### Example 5: Preparation of UV-QXL2 (combination crosslinker/UV-absorber tetrakis(dimethylsiloxy)silane/modified Tinuvin 326)

To a dry 250ml flask are weighed in order: tetrakis(dimethylsiloxysilane) (60.00 g, 0.183 moles), and Tinuvin 326 UV absorber (40.00g). The flask is equipped for reflux and the system is purged with nitrogen, and the mixture is heated at 70°C and stirred. After all Tinuvin UV absorber is dissolved, a Pt-catalyst (1.0µl) is added which was made from hydrogen hexachloroplatinum (IV) (4.2 g) dissolved in 46.0 g octanol. The mixture is heated and stirred for 48 hours at a temperature of 70° C. After cooling, the product is filtered through a 1µm PTFE filter. The combination crosslinker/UV-absorber, UV-QXL2, is a yellowish product. ¹H-NMR, 300MHz, gives no significant vinyl peaks between 5.1-6.1ppm.

### Example 6: Preparation and curing of the injectable polysiloxane material

Mixed end-blocker silicone polymer of refractive index 1.45 having a composition of 25 % w/w DVT-, 50 %w/w MVT- and 25 % w/w NVT-polysiloxanes, synthesized in example 3, is prepared for curing by formulating two equal parts. The quantities of the combined crosslinkers are adjusted so that mol-ratio Vinyl/SiH in the combination of the A and B formulations is at a maximum 1:1, for example in a range between about 1:0.5 and about 1:1. The amounts of catalyst and inhibitor are adjusted to give a gel time in a range between about 10 minutes and about 90 minutes at room temperature. Part A comprises 0.385 g of a platinum catalyst solution, comprising 5.000 (w/w) of the active compound Pt₂[(CH₂CHSi(Me)₂)₂O]₃ dissolved in a polymer produced according to Example 4, and 349.62 g of the silicone polymer. Part B comprises a mixture of 1.358 % (w/w) the 5-9 functional crosslinker HMS301 (Gelest Inc.), and 0.264 (w/w) the combination crosslinker/UV-absorber UV-QXL2 made according to Example 5, plus 0.011 % (w/w) of the inhibitor 1,3-divinyltetramethyldisiloxane (DVTMDS). Samples of Part A and Part B are weighed into three different cartridges, and A and B are mixed by means of static mixers. Curing is performed during 2.5 hours (9000 seconds) at 35.0°C. The curing rheology is screened using a Bohlin C-VOR rheometer at 35°C and a frequency of 1 Hz. At 9000 s, for the cured material, the complex shear G*, elastic shear G' and viscous shear moduli G" are determined (estimated) for the three cartridges:

**Table 1**

| | G* (9000) Pa | G'(9000) Pa | G"(9000) Pa |
|---|---|---|---|
| Cartridge 1 | 464 (404) | 348 | 306 |
| Cartridge 2 | 452 | 343 (353) | 294 (298) |
| Cartridge 3 | 486 | 371 | 315 |

Accordingly, all three cartridge samples demonstrate a desirable rheology with a complex shear modulus ≤ 600 Pa as was specified as for capsular filling lenses to undergo accommodation.

The specific embodiments and examples described herein are illustrious in nature only and are not intended to be limiting of the invention defined by the claims. Additional embodiments and examples of the various aspects of the invention defined by the claims and/or which are equivalent to the specific embodiments and examples set forth herein may be apparent to one of ordinary skill in the art and are included within the scope of the claimed invention.

## Claims

1. An injectable ophthalmic composition suitable for forming an intraocular lens in the capsular bag of an eye, comprising
(a) linear non-functional polysiloxanes,
(b) linear terminally functional polysiloxanes, and
(c) at least one crosslinker
wherein said functional polysiloxanes are a mixture of linear terminally mono-functional polysiloxanes, and linear terminally di-functional polysiloxanes.

2. An injectable ophthalmic composition according to claim 1, wherein the at least one crosslinker is a multifunctional crosslinker.

3. An injectable ophthalmic composition according to any one of claims 1-2, wherein the functionality of said terminally linear polysiloxanes is provided by functional unsaturated groups.

4. An injectable ophthalmic composition according to any one of claims 1-3, which composition is a thermocurable composition and which composition further comprises a catalyst, wherein said functional unsaturated groups of said linear terminally functional polysiloxanes are thermocurable.

5. An injectable ophthalmic composition according to claim 4, wherein said thermocurable functional unsaturated groups are vinyl groups.

6. An injectable ophthalmic composition according to claim 4, wherein said catalyst is a platinum complex

7. An injectable ophthalmic composition according to claim 6, wherein the catalyst is a complex between platinum and a siloxane-containing compound.

8. A composition according to claim 7, wherein the catalyst comprises a complex between platinum and divinyltetramethyldisiloxane.

9. An injectable ophthalmic composition according to any one of claims 1-3, which composition is a photocurable composition and said which composition further comprises a photoinitiator, optionally covalently bound to or incorporated in the crosslinker, wherein said functional unsaturated groups of said linear terminally functional polysiloxanes are photocurable.

10. An injectable ophthalmic composition according to claim 9, wherein said photocureable functional unsaturated groups are acrylic groups.

11. An injectable ophthalmic composition according to any one of the preceding claims,
wherein the molar ratio between the linear terminally di-functional polysiloxanes, the linear terminally mono-functional polysiloxanes and linear non-functional polysiloxanes is 0.5-1:1-2:0.5-1.

12. An injectable ophthalmic composition according to any one of the preceding claims,
wherein said linear terminally functional polysiloxanes and said linear non-functional polysiloxanes are essentially composed of the same monomer units and wherein the molar ratios of said monomer units is essentially the same.

13. An injectable ophthalmic composition according to claim 12, wherein said linear terminally functional polysiloxane and non-functional polysiloxanes comprise monomer units have the general formula of -RₐR_{b}-SiO-.

14. An injectable ophthalmic composition according to claim 13, wherein Rₐ and R_{b} are the same or different substituted or unsubstituted alkyl or aryl groups which are bonded to the silicone atom and wherein one of said alkyl or aryl groups is substituted with at least one fluorine atom.

15. An injectable ophthalmic composition according to any one of claims 13-14, wherein Rₐ in at least one of said monomer units is fluoroalkyl and wherein R_{b} in that monomer unit is alkyl.

16. An injectable ophthalmic composition according to any one of claims 13-15, wherein said Rₐ is 3,3,3-trifluoroalkyl.

17. An injectable ophthalmic composition according to any one of claims 13-16, wherein the obtained polysiloxanes have a specific gravity greater than about 1.

18. An injectable ophthalmic composition according to claim 12, wherein said linear terminally functional and non-functional polysiloxanes are derived from three different siloxane monomers having the general formula (R₁R₂SiO)₁, (R₃R₄SiO)ₘ and (R₅R₆SiO)ₙ.

19. An injectable ophthalmic composition according to claim 18, wherein one of the three monomers has a specific gravity greater than about 1.0 and wherein R₁ and R₂ are the same or different substituted or unsubstituted C₁₋₆ alkyl groups, R₃ and R₄ are selected among the same or different substituted aryl and C₁₋₆ alkyl groups and R₅ and R₆ are selected among fluoroalkyl and alkyl groups and wherein 1 is in the molar fraction range of 0 to 0.95, m is in the molar fraction range of 0 to 0.7 and n is in the molar fraction range of 0 to 0.65.

20. An injectable ophthalmic composition according to any one of claims 18-19, wherein R₁ and R₂ are methyl, R₃ is phenyl and R₄ is phenyl or methyl, R₅ is trifluoropropyl and R₆ is methyl.

21. An injectable ophthalmic composition according to any one of claims 18-20, wherein said linear terminally functional and non-functional polysiloxanes are random terpolymers.

22. An injectable ophthalmic composition according to any one of the preceding claims,
wherein said linear terminally functional and non-functional polysiloxanes are poly(dimethyl-co-diphenyl-co-trifluoropropylmethyl) having a molecular weight, Mₙ, in the range of 10,000 to 25,000 D and a refractive index of 1.40 to 1.45 and a density greater than about 1.

23. An injectable ophthalmic composition according to any one of claims 1-22 wherein said multifunctional crosslinker is a siloxane copolymer and wherein said multifunctional crosslinker has more than four reactive groups.

24. An injectable ophthalmic composition according to claim 23 wherein said multifunctional copolymer siloxane crosslinker comprises organohydrogen siloxane and dialkyl siloxane.

25. An injectable ophthalmic composition according to claim 24, wherein said crosslinker is a methylhydrosiloxane dimethylsiloxane copolymer.

26. An injectable ophthalmic composition according to any one of the preceding claims, wherein said composition further comprises another crosslinker.

27. An injectable ophthalmic composition according to claim 26, wherein said crosslinker is a multifunctional crosslinker.

28. An injectable ophthalmic composition according to any one of claims 26-27, wherein said crosslinker has more than three but not more than four reactive groups.

29. An injectable ophthalmic composition according to any one of claims 26-28, wherein said crosslinker is an organohydrogen siloxane.

30. An injectable ophthalmic composition according to claim 29, wherein said crosslinker is tetrakis(dimethylsiloxy)silane.

31. An injectable ophthalmic composition according to claim 30, wherein said tetrakis(dimethylsiloxy)-silane has been bonded to an UV-absorber.

32. An accommodating intraocular lens made from the injectable ophthalmic composition according to any one of claims 1-31.

## Patentansprüche

1. Injizierbare ophthalmische Zusammensetzung, geeignet für die Bildung einer intraokularen Linse im Kapselsack eines Auges, umfassend
(a) lineare, nicht funktionelle Polysiloxane,
(b) lineare endständig funktionelle Polysiloxane und
(c) mindestens einen Vernetzer
wobei die funktionellen Polysiloxane eine Mischung aus linearen endständig monofunktionellen Polysiloxanen und linearen endständig difunktionellen Polysiloxanen sind.

2. Injizierbare ophthalmische Zusammensetzung nach Anspruch 1, wobei der mindestens eine Vernetzer ein multifunktioneller Vernetzer ist.

3. Injizierbare ophthalmische Zusammensetzung nach einem der Ansprüche 1-2, wobei die Funktionalität der endständig linearen Polysiloxane durch funktionelle ungesättigte Gruppen bereitgestellt wird.

4. Injizierbare ophthalmische Zusammensetzung nach einem der Ansprüche 1-3, die eine wärmehärtbare Zusammensetzung ist und weiter einen Katalysator umfasst, wobei die funktionellen ungesättigten Gruppen der linearen endständig funktionellen Polysiloxane wärmehärtbar sind.

5. Injizierbare ophthalmische Zusammensetzung nach Anspruch 4, wobei die wärmehärtbaren funktionellen ungesättigten Gruppen Vinylgruppen sind.

6. Injizierbare ophthalmische Zusammensetzung nach Anspruch 4, wobei der Katalysator ein Platinkomplex ist.

7. Injizierbare ophthalmische Zusammensetzung nach Anspruch 6, wobei der Katalysator ein Komplex zwischen Platin und einer siloxanhaltigen Verbindung ist.

8. Zusammensetzung nach Anspruch 7, wobei der Katalysator einen Komplex zwischen Platin und Divinyltetramethyldisiloxan umfasst.

9. Injizierbare ophthalmische Zusammensetzung nach einem der Ansprüche 1-3, die eine lichthärtbare Zusammensetzung ist und weiter einen Photoinitiator umfasst, der optional kovalent an den Vernetzer gebunden oder darin eingeschlossen ist, wobei die funktionellen ungesättigten Gruppen der linearen endständig funktionellen Polysiloxane lichthärtbar sind.

10. Injizierbare ophthalmische Zusammensetzung nach Anspruch 9, wobei die lichthärtbaren funktionellen ungesättigten Gruppen Acrylgruppen sind.

11. Injizierbare ophthalmische Zusammensetzung nach einem der vorhergehenden Ansprüche, wobei das Molverhältnis zwischen den linearen endständig difunktionellen Polysiloxanen, den linearen endständig monofunktionellen Polysiloxanen und den linearen nicht funktionellen Polysiloxanen 0,5-1:1-2:0,5-1 ist.

12. Injizierbare ophthalmische Zusammensetzung nach einem der vorhergehenden Ansprüche, wobei die linearen endständig funktionellen Polysiloxane und die linearen nicht funktionellen Polysiloxane im Wesentlichen aus den gleichen Monomereinheiten zusammengesetzt sind, und wobei die Molarverhältnisse der Monomereinheiten im Wesentlichen die gleichen sind.

13. Injizierbare ophthalmische Zusammensetzung nach Anspruch 12, wobei das lineare endständig funktionelle Polysiloxan und die nicht funktionellen Polysiloxane Monomereinheiten umfassen, die die allgemeine Formel - RₐR_{b}-SiO- haben.

14. Injizierbare ophthalmische Zusammensetzung nach Anspruch 13, wobei Rₐ and R_{b} die gleichen oder verschiedene substituierte oder nicht substituierte Alkyl- oder Arylgruppen sind, die an das Siliziumatom gebunden sind, und wobei eine der Alkyl- oder Arylgruppen durch mindestens ein Fluoratom substituiert ist.

15. Injizierbare ophthalmische Zusammensetzung nach einem der Ansprüche 13-14, wobei Rₐ in mindestens einem der Monomereinheiten Fluoralkyl ist und wobei R_{b} in dieser Monomereinheit Alkyl ist.

16. Injizierbare ophthalmische Zusammensetzung nach einem der Ansprüche 13-15, wobei Rₐ für 3,3,3-Trifluoralkyl steht.

17. Injizierbare ophthalmische Zusammensetzung nach einem der Ansprüche 13-16, wobei die erhaltenen Polysiloxane eine relative Dichte haben, die größer als 1 ist.

18. Injizierbare ophthalmische Zusammensetzung nach Anspruch 12, wobei die linearen endständig funktionellen und nicht funktionellen Polysiloxane von drei verschiedenen Siloxanmonomeren mit der allgemeinen Formel (R₁R₂SiO)₁, (R₃R₄SiO)ₘ und (R₅R₆SiO)ₙ abgeleitet sind

19. Injizierbare ophthalmische Zusammensetzung nach Anspruch 18, wobei eines der drei Monomere eine relative Dichte aufweist, die größer als ungefähr 1,0 ist, und wobei R₁ und R₂ die gleichen oder verschiedene substituierte oder unsubstituierte C₁₋₆-Alkylgruppen sind, R₃ und R₄ aus den gleichen oder verschiedenen substituierten Aryl- und C₁₋₆-Alkylgruppen ausgewählt werden und R₅ and R₆ unter Fluoralkyl- und Alkylgruppen ausgewählt werden, und wobei 1 im Molenbruchbereich von 0 bis 0,95, m im Molenbruchbereich von 0 bis 0,7 und n im Molenbruchbereich von 0 bis 0,65 liegt.

20. Injizierbare ophthalmische Zusammensetzung nach einem der Ansprüche 18-19, wobei R₁ und R₂ Methyl sind, R₃ Phenyl ist und R₄ Phenyl oder Methyl ist, R₅ Trifluoropropyl und R₆ Methyl ist.

21. Injizierbare ophthalmische Zusammensetzung nach einem der Ansprüche 18-20, wobei die linearen endständig funktionellen und nicht funktionellen Polysiloxane statistische Terpolymere sind.

22. Injizierbare ophthalmische Zusammensetzung nach einem der vorhergehenden Ansprüche, wobei die linearen endständig funktionellen und nicht funktionellen Polysiloxane Poly(dimethyl-co-diphenyl-co-trifluoropropylmethyl) mit einer relativen Molekülmasse, Mₙ, im Bereich von 10,000 bis 25,000 D und einem Brechungsindex von 1,40 bis 1,45 und einer Dichte sind, die größer als ungefähr 1 ist.

23. Injizierbare ophthalmische Zusammensetzung nach einem der Ansprüche 1-22, wobei der multifunktionelle Vernetzer ein Siloxancopolymer ist, und wobei der multifunktionelle Vernetzer mehr als vier reaktive Gruppen aufweist.

24. Injizierbare ophthalmische Zusammensetzung nach Anspruch 23, wobei der multifunktionelle Copolymer-Siloxan-Vernetzer Organohydrogensiloxan und Dialkylsiloxan umfasst.

25. Injizierbare ophthalmische Zusammensetzung nach Anspruch 24, wobei der Vernetzer ein Methylhydrosiloxan-Dimethylsiloxan-Copolymer ist.

26. Injizierbare ophthalmische Zusammensetzung nach einem der vorhergehenden Ansprüche, wobei die Zusammensetzung außerdem einen weiteren Vernetzer umfasst.

27. Injizierbare ophthalmische Zusammensetzung nach Anspruch 26, wobei der Vernetzer ein multifunktioneller Vernetzer ist.

28. Injizierbare ophthalmische Zusammensetzung nach einem der Ansprüche 26-27, wobei der Vernetzer mehr als drei aber nicht mehr als vier reaktive Gruppen aufweist.

29. Injizierbare ophthalmische Zusammensetzung nach einem der Ansprüche 26-28, wobei der Vernetzer ein Organohydrogensiloxan ist.

30. Injizierbare ophthalmische Zusammensetzung nach Anspruch 29, wobei der Vernetzer Tetrakis(dimethylsiloxy)silan ist.

31. Injizierbare ophthalmische Zusammensetzung nach Anspruch 30, wobei das Tetrakis(dimethylsiloxy)silan an einen UV-Absorber gebunden wurde.

32. Sich anpassende intraokulare Linse, hergestellt aus der injizierbaren ophthalmischen Zusammensetzung nach einem der Ansprüche 1-31.

## Revendications

1. Composition ophtalmique injectable adaptée pour former une lentille intraoculaire dans le sac capsulaire d'un oeil, comprenant
(a) des polysiloxanes non fonctionnels linéaires
(b) des polysiloxanes terminalement fonctionnels linéaires et
(c) au moins un agent de réticulation
dans laquelle lesdits polysiloxanes fonctionnels sont un mélange de polysiloxanes terminalement monofonctionnels linéaires et de polysiloxanes terminalement difonctionnels linéaires.

2. Composition ophtalmique injectable selon la revendication 1, dans laquelle l'au moins un agent de réticulation est un agent de réticulation multifonctionnel.

3. Composition ophtalmique injectable selon l'une quelconque des revendications 1 et 2, dans laquelle la fonctionnalité desdits polysiloxanes terminalement linéaires est fournie par des groupes insaturés fonctionnels.

4. Composition ophtalmique injectable selon l'une quelconque des revendications 1 à 3, laquelle composition est une composition thermodurcissable et laquelle composition comprend en outre un catalyseur, dans laquelle lesdits groupes insaturés fonctionnels desdits polysiloxanes terminalement fonctionnels linéaires sont thermodurcissables.

5. Composition ophtalmique injectable selon la revendication 4, dans laquelle lesdits groupes insaturés fonctionnels thermodurcissables sont des groupes vinyle.

6. Composition ophtalmique injectable selon la revendication 4, dans laquelle ledit catalyseur est un complexe de platine.

7. Composition ophtalmique injectable selon la revendication 6, dans laquelle le catalyseur est un complexe entre le platine et un composé contenant du siloxane.

8. Composition selon la revendication 7, dans laquelle le catalyseur comprend un complexe entre le platine et le divinyltétraméthyldisiloxane.

9. Composition ophtalmique injectable selon l'une quelconque des revendications 1 à 3, laquelle composition est une composition thermodurcissable et laquelle dite composition comprend en outre un photoinitiateur, lié éventuellement de manière covalente à l'agent de réticulation ou incorporé dans celui-ci, dans laquelle lesdits groupes insaturés fonctionnels desdits polysiloxanes terminalement fonctionnels linéaires sont photodurcissables.

10. Composition ophtalmique injectable selon la revendication 9, dans laquelle lesdits groupes insaturés fonctionnels photodurcissables sont des groupes acryliques.

11. Composition ophtalmique injectable selon l'une quelconque des revendications précédentes, dans laquelle le rapport molaire entre les polysiloxanes terminalement difonctionnels linéaires, les polysiloxanes terminalement monofonctionnels linéaires et les polysiloxanes non fonctionnels linéaires est de 0,5-1:1-2:0,5-1.

12. Composition ophtalmique injectable selon l'une quelconque des revendications précédentes, dans laquelle lesdits polysiloxanes terminalement fonctionnels linéaires et lesdits polysiloxanes non fonctionnels linéaires sont essentiellement composés des mêmes unités monomères et dans laquelle le rapport molaire desdites unités monomères est essentiellement le même.

13. Composition ophtalmique injectable selon la revendication 12, dans laquelle ledit polysiloxane terminalement fonctionnel linéaire et les polysiloxanes non fonctionnels comprennent des unités monomères qui ont la formule générale -RₐR_{b}-SiO-.

14. Composition ophtalmique injectable selon la revendication 13, dans laquelle Rₐ et R_{b} sont les groupes alkyles ou aryles substitués ou non substitués identiques ou différents qui sont liés à l'atome de silicium et dans laquelle l'un desdits groupes alkyle ou aryle est substitué avec au moins un atome de fluor.

15. Composition ophtalmique injectable selon l'une quelconque des revendications 13 et 14, dans laquelle Rₐ dans au moins l'une desdites unités monomères est un groupe fluoroalkyle et dans laquelle R_{b} dans cette unité monomère est alkyle.

16. Composition ophtalmique injectable selon l'une quelconque des revendications 13 à 15, dans laquelle ledit Rₐ est 3,3,3-trifluoroalkyle.

17. Composition ophtalmique injectable selon l'une quelconque des revendications 13 à 16, dans laquelle les polysiloxanes obtenus ont une gravité spécifique supérieure à 1 environ.

18. Composition ophtalmique injectable selon la revendication 12, dans laquelle lesdits polysiloxanes terminalement fonctionnels et non fonctionnels linéaires sont dérivés de trois monomères de siloxane différents ayant la formule générale (R₁R₂SiO)₁, (R₃R₄SiO)ₘ et (R₅R₆SiO)ₙ.

19. Composition ophtalmique injectable selon la revendication 18, dans laquelle l'un des trois monomères a une gravité spécifique supérieure à environ 1,0 et dans laquelle R₁ et R₂ sont les groupes alkyles en C₁-C₆ substitués ou non substitués identiques ou différents, R₃ et R₄ sont choisis parmi les groupes aryle et alkyle en C₁-C₆ substitués identiques ou différents et R₅ et R₆ sont choisis parmi les groupes fluoroalkyle et alkyle et dans laquelle 1 est dans la plage de fraction molaire de 0 à 0,95, m est dans la plage de fraction molaire de 0 à 0,7 et n est dans la plage de fraction molaire de 0 à 0,65.

20. Composition ophtalmique injectable selon l'une quelconque des revendications 18 et 19, dans laquelle R₁ et R₂ sont un groupe méthyle, R₃ est un groupe phényle et R₄ est un groupe phényle ou méthyle, R₅ est un groupe trifluoropropyle et R₆ est un groupe méthyle.

21. Composition ophtalmique injectable selon l'une quelconque des revendications 18 à 20, dans laquelle lesdits polysiloxanes terminalement fonctionnels et non fonctionnels linéaires sont des terpolymères aléatoires.

22. Composition ophtalmique injectable selon l'une quelconque des revendications précédentes, dans laquelle lesdits polysiloxanes terminalement fonctionnels et non fonctionnels linéaires sont du poly(diméthyl-co-diphényl-co-trifluoropropylméthyle) ayant une masse moléculaire, Mₙ, dans la plage de 10 000 à 25 000 D et un indice de réfraction de 1,40 à 1,45 et une densité supérieure à environ 1.

23. Composition ophtalmique injectable selon l'une quelconque des revendications 1 à 22, dans laquelle ledit agent de réticulation multifonctionnel est un copolymère siloxane et dans laquelle ledit agent de réticulation multifonctionnel possède plus de quatre groupes réactifs.

24. Composition ophtalmique injectable selon la revendication 23, dans laquelle ledit agent de réticulation de siloxane copolymère multifonctionnel comprend du siloxane organohydrogéné et du siloxane de dialkyle.

25. Composition ophtalmique injectable selon la revendication 24, dans laquelle ledit agent de réticulation est un copolymère de méthylhydrosiloxane et de diméthylsiloxane.

26. Composition ophtalmique injectable selon l'une quelconque des revendications précédentes, dans laquelle ladite composition comprend en outre un autre agent de réticulation.

27. Composition ophtalmique injectable selon la revendication 26, dans laquelle ledit agent de réticulation est un agent de réticulation multifonctionnel.

28. Composition ophtalmique injectable selon l'une quelconque des revendications 26 et 27, dans laquelle ledit agent de réticulation a plus de trois mais pas plus de quatre groupes réactifs.

29. Composition ophtalmique injectable selon l'une quelconque des revendications 26 à 28, dans laquelle ledit agent de réticulation est un siloxane organohydrogéné.

30. Composition ophtalmique injectable selon la revendication 29, dans laquelle ledit agent de réticulation est un tétrakis(diméthylsiloxy)silane.

31. Composition ophtalmique injectable selon la revendication 30, dans laquelle ledit tétrakis(diméthylsiloxy)-silane a été lié à un absorbeur d'UV.

32. Lentille intraoculaire d'accomodation fabriquée à partir de la composition ophtalmique injectable selon l'une quelconque des revendications 1 à 31.
